# EUROPEAN PATENT SPECIFICATION

(11) **EP 1 301 614 B1**
(45) Date of publication and mention of the grant of the patent: **29.11.2006**
(21) Application number: 01953199.5
(22) Date of filing: 20.07.2001
(51) Int. Cl.: C12N 15/86, C12N 5/10, C12N 1/21, C07K 14/025, A61K 39/12

(54) **CODON-OPTIMIZED PAPILLOMA VIRUS SEQUENCES**
KODON-OPTIMIERTE PAPILLOMAVIRUSSEQUENZEN
SEQUENCES DE PAPILLOMAVIRUS A CODON OPTIMISE

(30) Priority: 21.07.2000 GB 0017990; 20.10.2000 GB 0025802
(43) Date of publication of application: 16.04.2003
(73) Proprietor: GLAXO GROUP LIMITED, Greenford, Middlesex UB6 ONN (GB)
(72) Inventor: ERTL, Peter F., Stevenage, Hertfordshire SG1 2NY (GB); GOUGH, Gerald W., Stevenage, Hertfordshire SG1 2NY (GB); RING, Christopher J., Stevenage, Hertfordshire SG1 2NY (GB); WALCOTT, Sarah, Marina GlaxoSmithKline, Hertfordshire SG1 2NY (GB)
(74) Representative: Giddings, Peter John
(86) International application number: PCT/GB2001/003290
(87) International publication number: WO 2002/008435

(56) References cited:
- EP-A- 0 523 395
- WO-A-01/14416
- WO-A-92/11290
- ZHOU JIAN ET AL: "Papillomavirus capsid protein expression level depends on the match between codon usage and tRNA availability" JOURNAL OF VIROLOGY, THE AMERICAN SOCIETY FOR MICROBIOLOGY, US, vol. 73, no. 6, June 1999 (1999-06), pages 4972-4982, XP002164427 ISSN: 0022-538X
- DARTMANN K ET AL: "The nucleotide sequence and genome organization of Human Papilloma virus type 11" VIROLOGY, RAVEN PRESS, NEW YORK, NY, US, vol. 151, 1986, pages 124-130, XP002170550 ISSN: 0042-6822
- SCHWARZ E ET AL: "DNA sequence and genome organization of genital human papillomavirus type 6b" EMBO JOURNAL, IRL PRESS, EYNSHAM, GB, vol. 2, no. 12, 1983, pages 2341-2348, XP002101771 ISSN: 0261-4189
- GOUY M ET AL: "CODON USAGE IN BACTERIA: CORRELATION WITH GENE EXPRESSIVITY" NUCLEIC ACIDS RESEARCH, IRL PRESS LTD., OXFORD, GB, vol. 10, no. 22, 1982, pages 7055-7074, XP002911021 ISSN: 0305-1048
- HALE R S ET AL: "CODON OPTIMIZATION OF THE GENE ENCODING A DOMAIN FROM HUMAN TYPE 1 NEUROFIBROMIN PROTEIN RESULTS IN A THREEFOLD IMPROVEMENT IN EXPRESSION LEVEL IN ESCHERICHIA COLI" PROTEIN EXPRESSION AND PURIFICATION, ACADEMIC PRESS, US, vol. 12, March 1998 (1998-03), pages 185-188, XP001030791 ISSN: 1046-5928 cited in the application
- HAN R. ET AL: 'Protection of rabbits from viral challenge by gene gun-based intracutaneous vaccination with a combination of cottontail rabbit papillomavirus E1, E2, E6 and E7.' J. VIROL. vol. 73, no. 8, August 1999, pages 7039 - 7043

## Description

The present invention relates to methods and compositions useful in the treatment and prevention of human papilloma virus infections and the symptoms and diseases associated therewith.

Papilloma virus infections have been observed in a variety of species, including sheep, dogs, rabbits, monkeys, cattle and humans. Human papilloma viruses (HPV) have been classified into more than 80 types (Epidemiology and Biology of Cervical Cancer. Seminars in Surgical Oncology 1999 16:203-211). New (novel) types are defined as those where the L1 gene displays less than 90% sequence identity to L1 sequences from previously identified types, whilst a sub-type displays between 90% and 98% L1 sequence identity, and a variant more than 98% sequence identity (to the prototypical (parent) type). Papilloma viruses generally infect epithelia, but the different HPV types cause distinct diseases. For example, types 1-4, 7, 10 and 26-29 cause benign cutaneous warts, types 16, 18, 31, 33, 35, 39, 45, 51, 52, 56, 58, 59, and 68 are associated with cervical cancers and types 6 and 11 are implicated in genital warts (non-malignant condylomata of the genital tract).

The majority of genital warts (>90%) contain HPV genotypes 6 and 11. Whilst HPV-6 is the most prevalent genotype identified in single infections, both HPV-6 and HPV-11 may occasionally occur in the same lesion. Warts generally occur in several sites in infected individuals and more than 60% of patients with partners having condyloma (genital warts) develop lesions, with an average incubation time of 3 months. A range of treatment options are currently available. However, they rely upon excision or ablation and/or the use of topical gels and creams. They are not pain free, they may require frequent clinic visits, and efficacy is highly variable. Disease recurrence remains a significant problem for the effective management of this disease.

Genital warts may regress spontaneously and cell mediated immunity appears to be the primary event responsible for wart regression. The high spontaneous regression rates indicate that host cellular immunity can resolve clinical disease and make immune-therapy intervention an option for treatment or prevention of genital warts. The goals for disease management are for a virus-specific therapy that is pain free, requires a minimum of clinic visits, has high disease resolution rates and which reduces/minimises disease recurrence.

HPV has proven difficult to grow in tissue culture, so there is no traditional live or attenuated viral vaccine. Development of an HPV vaccine has also been slowed by the lack of a suitable animal model in which the human virus can be studied. This is because the viruses are highly species specific, so it is not possible to infect an immunocompetent animal with a human papilloma virus, as would be required for safety testing before a vaccine was first tried in humans.

Papilloma viruses have a DNA genome which encodes "early" and "late" genes designated E1 to E7, L1 and L2. The early gene sequences have been shown to have functions relating to viral DNA replication and transcription, evasion of host immunity, and alteration of the normal host cell cycle and other processes. For example the E1 protein is an ATP-dependent DNA helicase and is involved in initiation of the viral DNA replication process whilst E2 is a regulatory protein controlling both viral gene expression and DNA replication. Through its ability to bind to both E1 and the viral origin of replication, E2 brings about a local concentration of E1 at the origin, thus stimulating the initiation of viral DNA replication. The E4 protein appears to have a number of poorly defined functions but amongst these may be binding to the host cell cytoskeleton, whilst E5 appears to delay acidification of endosomes resulting in increased expression of EGF receptor at the cell surface and both E6 and E7 are known to bind cell proteins p53 and pRB respectively. The E6 and E7 proteins form HPV types associated with cervical cancer are known oncogenes. L1 and L2 encode the two viral structural (capsid) proteins.

Historically, vaccines have been seen as a way to prevent infection by a pathogen, priming the immune system to recognise the pathogen and neutralise it should an infection occur. The vaccine includes one or more antigens from the pathogen, commonly the entire organism, either killed or in a weakened (attenuated) form, or selected antigenic peptides from the organism. When the immune system is exposed to the antigen(s), cells are generated which retain an immunological "memory" of it for the lifetime of the individual. Subsequent exposure to the same antigen (e.g. upon infection by the pathogen) stimulates a specific immune response which results in elimination or inactivation of the infectious agent.

There are two arms to the immune response: a humoral (antibody) response and a cell-mediated response. Protein antigens derived from pathogens that replicate intracellularly (viruses and some bacteria) are processed within the infected host cell releasing short peptides which are subsequently displayed on the infected cell surface in association with class I major histocompatability (MHC I) molecules. When this associated complex of MHC I and peptide is contacted by antigen-specific CD8+ T-cells the T-cell is activated, acquiring cytotoxic activity. These cytotoxic T-cells (CTLs) can lyse infected host cells, so limiting the replication and spread of the infecting pathogen. Another important arm of the immune response is controlled by CD4+ T-cells. When antigen derived from pathogens is released into the extracellular milieu they may be taken up by specialised antigen-presenting cells (APCs) and displayed upon the surface of these cells in association with MHC II molecules. Recognition of antigen in this complex stimulates CD4+ T-cells to secrete soluble factors (cytokines) which regulate the effector mechanisms of other T-cells. Antibody is produced by B-cells. Binding of antigen to secreted antibody may neutralise the infectivity of a pathogen and binding of antigen to membrane-bound antibody on the surface of B-cells stimulates division of the B-cell so amplifying the B-cell response. In general, both antibody and cell-mediated immune responses (CD8+ and CD4+) are required to control infections by pathogens.

It is believed that it may be possible to harness the immune system, even after infection by a pathogen, to control or resolve the infection by inactivation or elimination of the pathogen. Such immune therapies (also known as "therapeutic" vaccines or immunotherapeutics) would ideally require a cell-mediated response to be effective, although both humoral and cell-mediated immune responses may be evoked.

It has been demonstrated (Benvenisty, N and Reshaf, L. PNAS 83 9551-9555) that inoculation of mice with calcium phosphate precipitated DNA results in expression of the peptides encoded by the DNA. Subsequently, intramuscular injection into mice of plasmid DNA which had not been precipitated was shown to result in uptake of the DNA into the muscle cells and expression of the encoded protein. Because expression of the DNA results in production of the encoded pathogen proteins within the host's cells, as in a natural infection, this mechanism can stimulate the cell-mediated immune response required for immune therapies or therapeutic vaccination, so a DNA-based drug could be applied as a prophylactic vaccine or as an immune therapy. DNA vaccines are described in WO90/11092 (Vical, Inc.).

DNA vaccination may be delivered by mechanisms other than intra-muscular injection. For example, delivery into the skin takes advantage of the fact that immune mechanisms are highly active in tissues that are barriers to infection such as skin and mucous membranes. Delivery into skin could be via injection, via jet injector (which forces a liquid into the skin, or underlying tissues including muscles, under pressure) or via particle bombardment, in which the DNA may be coated onto particles of sufficient density to penetrate the epithelium (US Patent No. 5371015). For example, the nucleotide sequences may be incorporated into a plasmid which is coated on to gold beads which are then administered under high pressure into the epidermis, such as, for example, as described in Haynes *et al* J. Biotechnology 44: 37-42 (1996). Projection of these particles into the skin results in direct transfection of both epidermal cells and epidermal Langerhan cells. Langerhan cells are antigen presenting cells (APC) which take up the DNA, express the encoded peptides, and process these for display on cell surface MHC proteins. Transfected Langerhan cells migrate to the lymph nodes where they present the displayed antigen fragments to lymphocytes, evoking an immune response. Very small amounts of DNA (less than 1µg, often less than 0.5µg) are required to induce an immune response via particle mediated delivery into skin and this contrasts with the milligram quantities of DNA known to be required to generate immune responses subsequent to direct intramuscular injection.

The expression and detection of HPV proteins in transfected mammalian cells such as HeLa, 293, or CHO cells has often proved difficult and so for biochemical and immunological studies requiring detectable expression of proteins, or quantities of pure proteins the E.coli, Baculovirus or Yeast protein expression systems are often used. In these systems the yields of protein are adequate making functional analysis and purification and subsequent biochemical and immunological studies practicable. However, direct protein detection methods (e.g. Western blotting) typically fail to detect E1 protein expression in transfected mammalian cells even when vectors with strong promoters such as CMV or SV40 are used. Methods designed to increase E1 protein expression in mammalian cells include cloning the 5' flanking sequences alongside the E1 gene (Remm et al. J.Virol 1999 **73,** 3062-3070) and amplification of the transfected E1 plasmid vector after transfection (Zou et al. J.Virol 1998 **72,** 3436-3441). Amplification of the input vector plasmid by replication after transfection has the net effect of increasing the E1 gene copy number in the cell, hence boosting protein levels and so facilitating detection of protein (by Western blotting). Because E1 protein expression and detection is problematic in mammalian cells, several authors have resorted to detecting expression of the protein by in vitro transcription-translation with ³⁵S-labelled methionine using the rabbit reticulocyte system (Promega), (Gopalakrishnan et al. Virology 1999 **256**, 330-339., Safari et al. Virology 1995 **211**, 385-396). However, this is a cell-free system and it requires the use of a modified promoter which contains the binding sequence for the RNA polymerase from phage T7.

E1 protein expression has additionally been detected indirectly, via detection of the in vitro DNA replication of a plasmid containing an HPV origin of DNA replication. Detection of this replicated origin containing plasmid acts as a surrogate for E1 (and E2) protein expression (Gopalakrishnan et al, Virology 1999 **256**, 330-339., Lu et al J.Virol 1993 **67**, 7131-7139 and Del Vecchio et al J.Virol 1992 **66**, 5949-5958). Both E1 and E2 are required for replication of the HPV origin, so transfection of mammalian cells with plasmids encoding both the E1 and E2 genes, plus a third plasmid carrying an HPV origin of DNA replication, will only result in replication of the origin carrying plasmid if expression of the E1 protein (and E2 protein) has been successful, albeit undetectable by the standard protein detection method (Western blotting).

The DNA code has 4 letters (A, T, C and G) and uses these to spell three letter "codons" which represent the amino acids of the proteins encoded in an organism's genes. The linear sequence of codons along the DNA molecule is translated into the linear sequence of amino acids in the protein(s) encoded by those genes. The code is highly degenerate, with 61 codons coding for the 20 natural amino acids and 3 codons representing "stop" signals. Thus, most amino acids are coded for by more than one codon - in fact several are coded for by four or more different codons.

Where more than one codon is available to code for a given amino acid, it has been observed that the codon usage patterns of organisms are highly non-random. Different species show a different bias in their codon selection and, furthermore, utilization of codons may be markedly different in a single species between genes which are expressed at high and low levels. This bias is different in viruses, plants, bacteria and mammalian cells, and some species show a stronger bias away from a random codon selection than others. For example, humans and other mammals are less strongly biased than certain bacteria or viruses. For these reasons, there is a significant probability that a mammalian gene expressed in E.coli or a viral gene expressed in mammalian cells will have an inappropriate distribution of codons for efficient expression. However, a gene with a codon usage pattern suitable for E.coli expression may also be efficiently expressed in humans. It is believed that the presence in a heterologous DNA sequence of clusters of codons which are rarely observed in the host in which expression is to occur, is predictive of low heterologous expression levels in that host.

There are several examples where changing codons from those which are rare in the host to those which are host-preferred ("codon optimisation") has enhanced heterologous expression levels, for example the BPV (bovine papilloma virus) late genes L1 and L2 have been codon optimised for mammalian codon usage patterns and this has been shown to give increased expression levels over the wild-type HPV sequences in mammalian (Cos-1) cell culture (Zhou et. al. J. Virol 1999. **73,** 4972-4982). In this work, every BPV codon which occurred more than twice as frequently in BPV than in mammals (ration of usage >2), and most codons with a usage ratio of >1.5 were conservatively replaced by the preferentially used mammalian codon. In WO97/31115, WO97/48370 and WO98/34640 (Merck & Co., Inc.) codon optimisation of HIV genes or segments thereof has been shown to result in increased protein expression and improved immunogenicity when the codon optimised sequences are used as DNA vaccines in the host mammal for which the optimisation was tailored. In this work, the sequences consist entirely of optimised codons (except where this would introduce an undesired restriction site, intron splice site etc.) because each viral codon is conservatively replaced with the optimal codon for the intended host.

In WO 011446 (Merck and Co., Inc) This same method was applied to HPV genes.

According to a first aspect, the present invention provides a polynucleotide sequence which encodes an HPV amino acid sequence of an HPV early antigen or fragment thereof, said polynucleotide being capable of raising an immune response when administered in-vivo wherein said polynucleotide has a codon usage coefficient (as defined below) of greater than 0.5 but less than 1.0 for a highly expressed human gene. Preferably the polynucleotide sequence is a DNA sequence. Ideally, the codon usage pattern of the polynucleotide sequence also resembles that of highly expressed E.coli genes. The polynucleotide sequence may be a DNA sequence, for example a double stranded DNA sequence. Preferably the polynucleotide sequence encodes a HPV polypeptide of an HPV type or sub-type associated with cervical cancer, benign cutaneous warts or genital warts, for example types, 1-4, 6, 7, 10, 11, 16, 18, 26-29, 31, 33, 35, 39, 45, 51, 52, 56, 58, 59, and 68, preferably types 6, 11, 16, 18, 33 or 45, which are associated particularly with cervical cancer and genital warts, most preferably HPV 11, 6a or 6b.

Accordingly, there is provided a synthetic gene comprising a plurality of codons together encoding an HPV amino acid sequence, wherein the selection of the possible codons used for encoding the amino acid sequence has been changed to resemble the optimal human codon usage such that the frequency of codon usage in the synthetic gene ore closely resembles that of highly expressed human genes than that of papilloma virus genes. Preferably the codon usage pattern is substantially the same as that for highly expressed human genes.

In certain embodiments, the encoded amino acid sequence is a wild-type HPV amino acid sequence. In alternative embodiments, the encoded amino acid sequence is a mutated HPV amino acid sequence comprising the wild-type sequence with amino acid changes, for example amino acid point mutations, sufficient to reduce or inactivate one or more of the natural biological functions of the polypeptide. The mutated amino acid sequence will desirably retain the immunogenicity of the wild-type polypeptide.

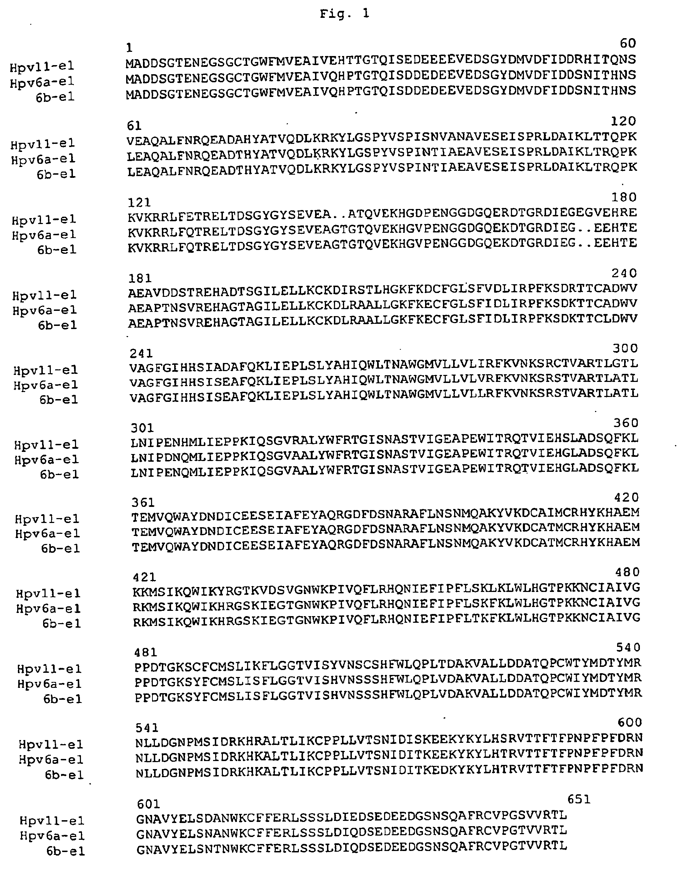

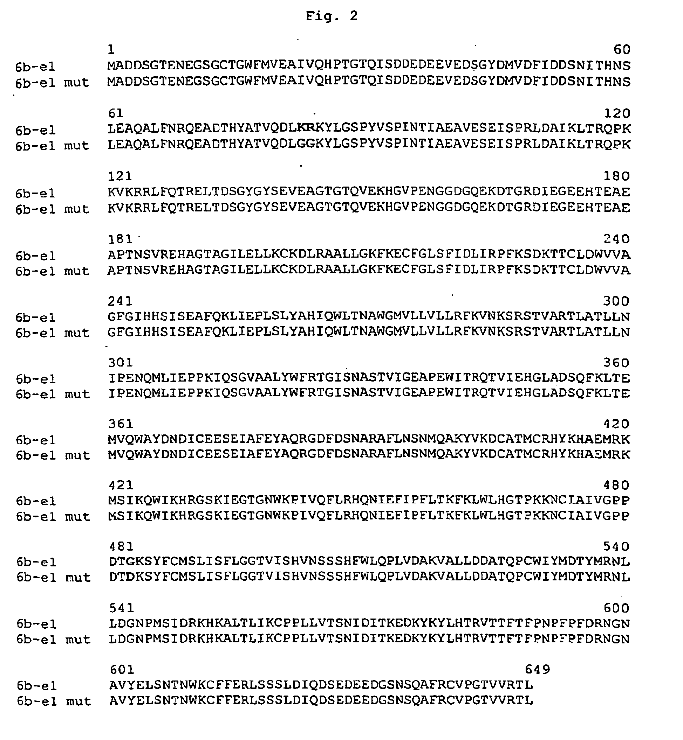

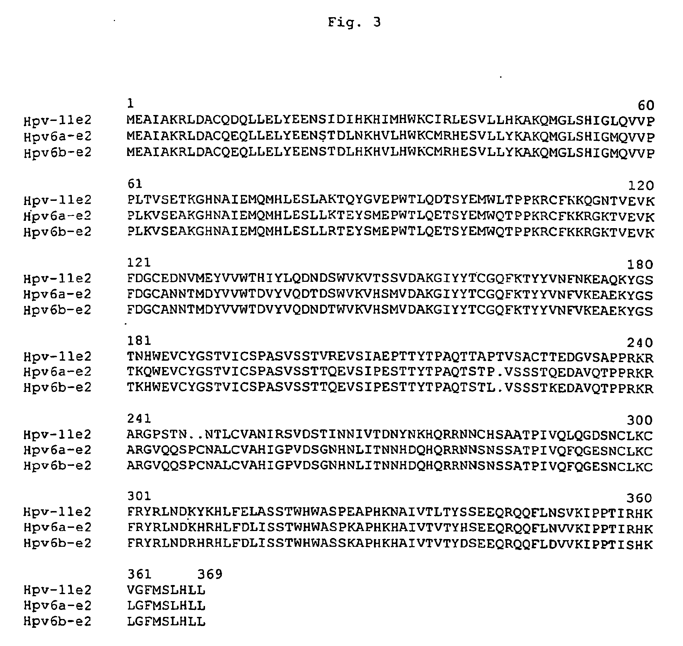

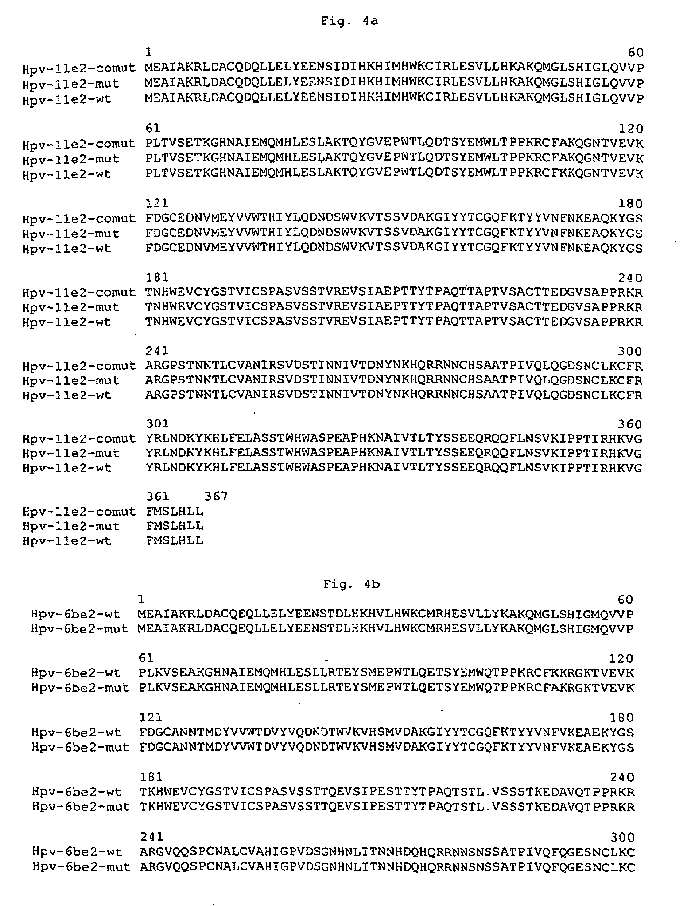

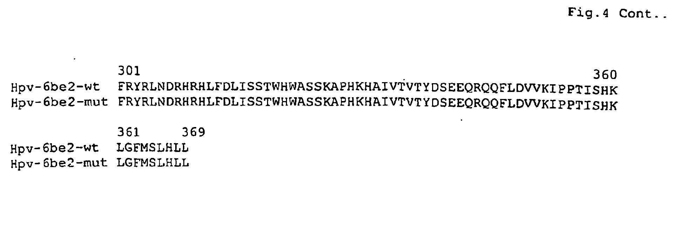

The encoded HPV polypeptide may comprise an early gene product such as E1, E2 or E7, or a fragment, analogue or fusion thereof. A polynucleotide of the invention may for example encode a fusion between two or more HPV early gene products, an HPV early gene product and an HPV late gene product for example L1 or L2, or a fragment, analogue or fusion thereof, or between two or more HPV late gene products, between one or more fragments of HPV gene products, or between an HPV gene product (or a fragment thereof) and a polypeptide derived from a source other than HPV, for example an adjuvant or targeting peptide, or polypeptide, such as an HBV core peptide. Fusions may be between HPV gene products derived from the same or different viral types or sub-types. Such fusions will desirably retain the immunogenicity of the fused polypeptide components. Preferably, the encoded HPV polypeptide comprises the whole or a part of an early gene product, most preferably E1 or E2. In one particular embodiment, the polynucleotide sequence encodes the wild-type E1 polypeptide of HPV 6b as set out in Fig. 1, or a fragment or analogue thereof. In alternative embodiments, the polynucleotide sequence may encode one or more of: the mutated HPV6b E1 amino acid sequence set out in Fig. 2; the witd-type E2 amino acid sequence (Fig. 3) of HPV 11 or of 6a or 6b; the mutated HPV6b E2 amino acid sequence of Fig. 4b; and the mutated HPV11 E2 sequence of Fig. 4a, or fragments, analogues or fusions thereof in which the encoded polypeptides retain immunogenicity.

According to the present invention, the codon usage pattern of the polynucleotide will preferably exclude codons with an RSCU value of less than 0.2 in highly expressed genes of the target organism. A relative synonymous codon usage (RSCU) value is the observed number of codons divided by the number expected if all codons for that amino acid were used equally frequently. A polynucleotide of the present invention will generally have a codon usage coefficient (as defined below) for highly expressed human genes of greater than 0.5 but less than 1. desirably the polynucleotide will also have a codon usage coefficient for highly expressed E.coli genes of greater than 0.5, preferably greater than 0.6, most preferably greater than 0.7.

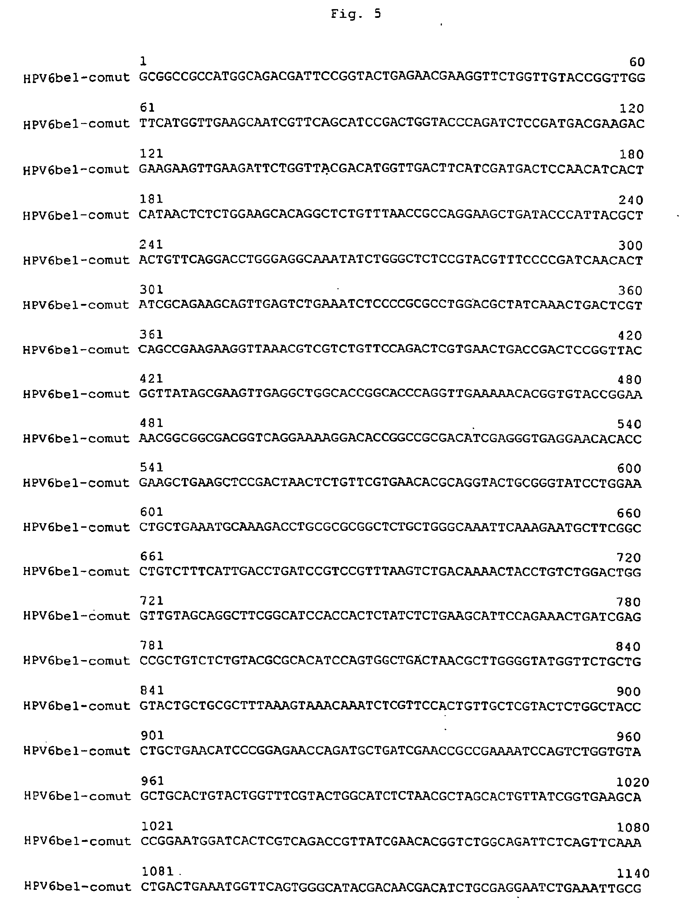

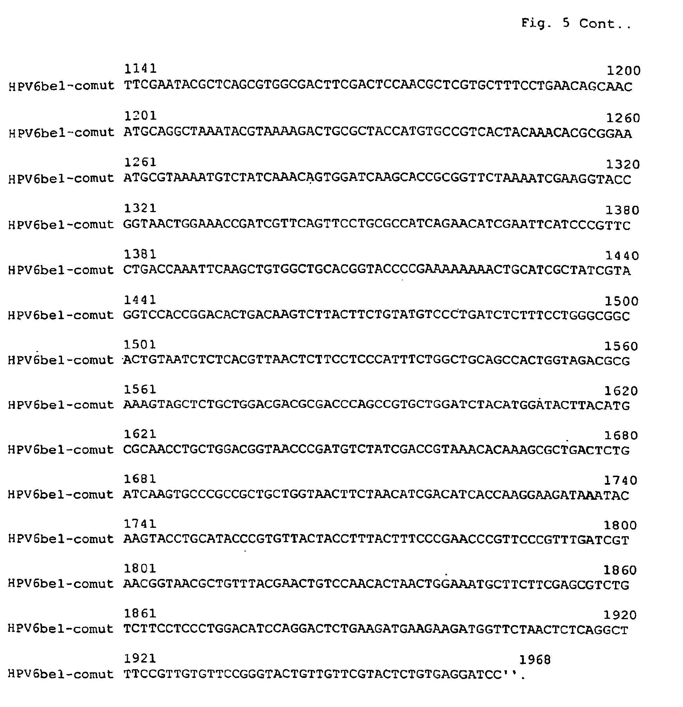

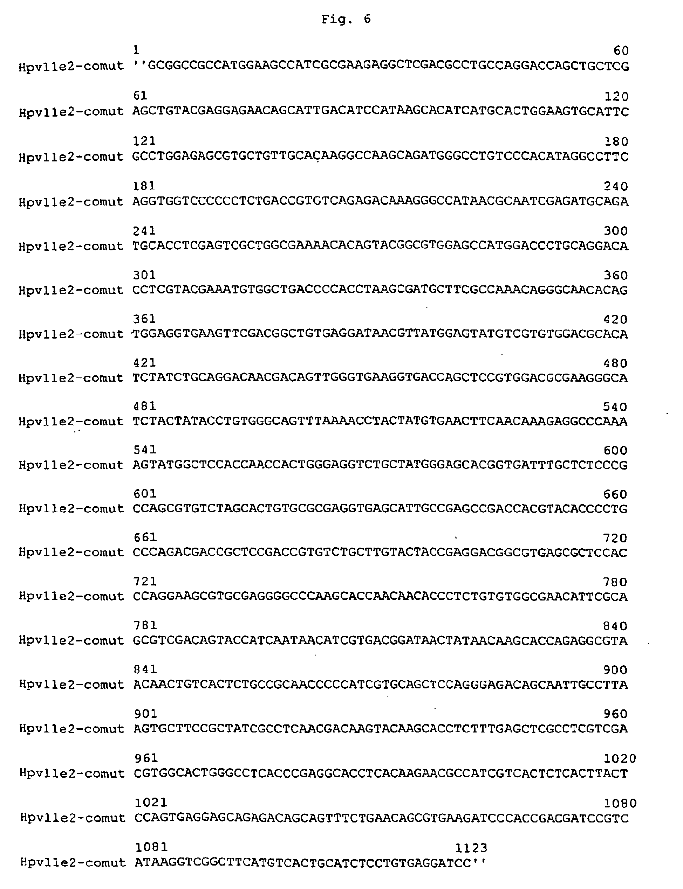

In one embodiment, the present invention provides a polynucleotide sequence as set out in Fig. 5 or Fig. 6, or a fragment or analogue thereof which maintains the codon usage pattern thereof. In a further embodiment, the present invention provides a polynucleotide sequence complementary to the sequence set out in Fig. 5 or Fig. 6.

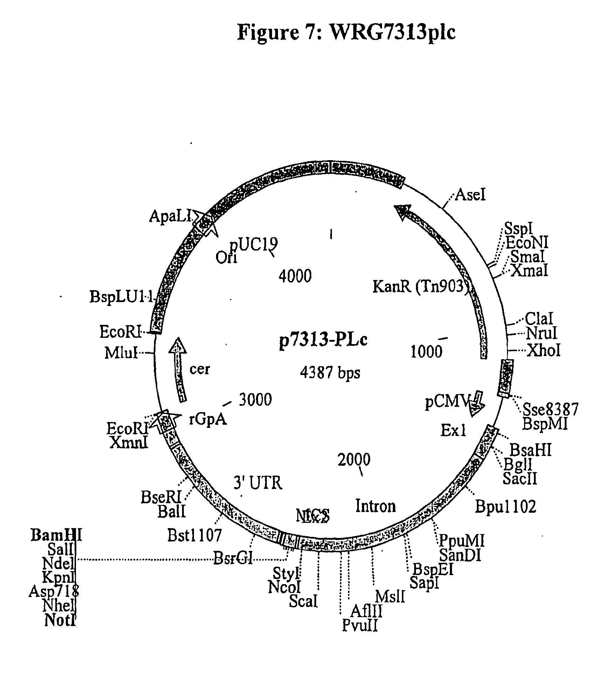

According to a second aspect of the invention, an expression vector is provided which comprises and is capable of directing the expression of a polynucleotide sequence according to the first aspect of the invention, encoding an HPV amino acid sequence wherein the codon usage pattern of the polynucleotide sequence resembles that of highly expressed human genes. The vector may be suitable for driving expression of heterologous DNA in bacterial insect or mammalian cells, particularly human cells. In one embodiment, the expression vector is p7313PLc (Fig.7).

According to a third aspect of the invention, a host cell comprising a polynucleotide sequence according to the first aspect of the invention, or an expression vector according the second aspect, is provided. The host cell may be bacterial, e.g. E.coli, mammalian, e.g. human, or may be an insect cell. Mammalian cells comprising a vector according to the present invention may be cultured cells transfected in vitro or may be transfected in vivo by administration of the vector to the mammal.

In a fourth aspect, the present invention provides a pharmaceutical composition comprising a polynucleotide sequence according to the first aspect of the invention. Preferably the composition comprises a DNA vector according to the second aspect of the present invention. In preferred embodiments the composition comprises a plurality of particles, preferably gold particles, coated with DNA comprising a vector encoding a polynucleotide sequence which encodes an HPV amino acid sequence, wherein the codon usage pattern of the polynucleotide sequence resembles that of highly expressed mammalian genes, particularly human genes. In alternative embodiments, the composition comprises a pharmaceutically acceptable excipient and a DNA vector according to the second aspect of the present invention. The composition may also include an adjuvant.

In a further aspect, the present invention provides a method of making a pharmaceutical composition including the step of altering the codon usage pattern of a wild-type HPV nucleotide sequence, or creating a polynucleotide sequence synthetically, to produce a sequence having a codon usage pattern resembling that of highly expressed mammalian genes and encoding a wild-type HPV amino acid sequence or a mutated HPV amino acid sequence comprising the wild-type sequence with amino acid changes sufficient to inactivate one or more of the natural functions of the polypeptide.

Also provided are the use of a polynucleotide according to the first aspect, or of a vector according to a second aspect of the invention, in the treatment or prophylaxis of an HPV infection, preferably an infection by an HPV type or sub-type associated with cervical cancer, benign cutaneous warts or genital warts, for example types, 1-4, 6, 7, 10, 11, 16, 18, 26-29, 31, 33, 35, 39, 45, 51, 52, 56, 58, 59, and 68. In certain embodiments, the invention provides the use of a polynucleotide according to the first aspect, or of a vector according to a second aspect of the invention, in the treatment or prophylaxis of an HPV infection of type 6, 11, 16, 18, 33 or 45, which are associated particularly with cervical cancer and genital warts, most preferably HPV 11, 6a or 6b. The invention also provides the use of a polynucleotide according to the first aspect, a vector according to the second aspect of the invention or a pharmaceutical composition according to the fourth aspect of the invention, in the treatment or prophylaxis of cutaneous (skin) warts, genital warts, atypical squamous cells of undetermined significance (ASCUS), cervical dysplasia, cervical intraepithelial neoplasia (CIN) or cervical cancer. Accordingly, the present invention also provides the use of a polynucleotide according to the first aspect, or of a vector according to the second aspect of the invention in making a medicament for the treatment or prophylaxis of an HPV infection of any one or more of types 1-4, 6, 7, 10, 11, 16, 18, 26-29, 31, 33, 35, 39, 45, 51, 52, 56, 58, 59, and 68, or any symptoms or disease associated therewith.

The present invention also provides methods of treating or preventing HPV infections, particularly infections by any one or more of HPV types 1-4, 6, 7, 10, 11, 16, 18, 26-29, 31, 33, 35, 39, 45, 51, 52, 56, 58, 59, and 68, or any symptoms or diseases associated therewith, comprising administering an effective amount of a polynucleotide according to the first aspect, a vector according to the second aspect or a pharmaceutical composition according to the fourth aspect of the invention. Administration of a pharmaceutical composition may take the form of one or more individual doses, for example in a "prime-boost" therapeutic vaccination regime. In certain cases the "prime" vaccination may be via particle mediated DNA delivery of a polynucleotide according to the present invention, preferably incorporated into a plasmid-derived vector and the "boost" by administration of a recombinant viral vector comprising the same polynucleotide sequence.

Throughout the present specification and the accompanying claims the words "comprise" and "include" and variations such as "comprises", "comprising", "includes" and "including" are to be interpreted inclusively. That is, these words are intended to convey the possible inclusion of other elements or integers not specifically recited, where the context allows.

The term "analogue" refers to a polynucleotide which encodes the same amino acid sequence as another polynucleotide of the present invention but which, through the redundancy of the genetic code, has a different nucleotide sequence whilst maintaining the same codon usage pattern, for example having the same codon usage coefficient or a codon usage coefficient within 0.1, preferably within 0.05 of that of the other polynucleotide.

The term "codon usage pattern" refers to the average frequencies for all codons in the nucleotide sequence, gene or class of genes under discussion (e.g. highly expressed mammalian genes). Codon usage patterns for mammals, including humans can be found in the literature (see e.g. Nakamura et.al. Nucleic Acids Research 1996, 24:214-215).

In the polynucleotides of the present invention, the codon usage pattern is altered from that typical of human papilloma viruses to more closely represent the codon bias of the target organism, e.g. E.coli or a mammal, especially a human. The "codon usage coefficient" is a measure of how closely the codon usage pattern of a given polynucleotide sequence resembles that of a target species. Codon frequencies can be derived from literature sources for the highly expressed genes of many species (see e.g. Nakamura et.al. Nucleic Acids Research 1996, **24**:214-215). The codon frequencies for each of the 61 codons (expressed as the number of occurrences occurrence per 1000 codons of the selected class of genes) are normalised for each of the twenty natural amino acids, so that the value for the most frequently used codon for each amino acid is set to 1 and the frequencies for the less common codons are scaled to lie between zero and 1. Thus each of the 61 codons is assigned a value of 1 or lower for the highly expressed genes of the target species. In order to calculate a codon usage coefficient for a specific polynucleotide, relative to the highly expressed genes of that species, the scaled value for each codon of the specific polynucleotide are noted and the geometric mean of all these values is taken (by dividing the sum of the natural logs of these values by the total number of codons and take the anti-log). The coefficient will have a value between zero and 1 and the higher the coefficient the more codons in the polynucleotide are frequently used codons. If a polynucleotide sequence has a codon usage coefficient of 1, all of the codons are "most frequent" codons for highly expressed genes of the target species.

Shorter polynucleotide sequences are within the scope of the invention. For example, a polynucleotide of the invention may encode a fragment of a HPV protein. A polynucleotide which encodes a fragment of at least 8, for example 8-10 amino acids or up to 20, 50, 60, 70, 80, 100, 150 or 200 amino acids in length is considered to fall within the scope of the invention as long as the polynucleotide has a codon usage pattern which resembles that of a highly expressed mammalian gene and the encoded oligo or polypeptide demonstrates HPV antigenicity. In particular, but not exclusively, this aspect of the invention encompasses the situation when the polynucleotide encodes a fragment of a complete HPV protein sequence and may represent one or more discrete epitopes of that protein.

The polynucleotides of the present invention show higher expression in E.coli and mammalian cells than corresponding wild-type sequences encoding the same amino acid sequences. Whilst not wishing to be bound by any theory, this is believed to be for at least two reasons. Firstly, having a codon usage pattern closer to that of the host cell, the sequences are more easily processed by the cell translation machinery. Secondly, as up to 30% of the nucleotide sequence (or more) is different from the wild-type sequence, sites which interfere with transcription or translation (such as protein binding sites) will have been removed or altered.

In some embodiments, polynucleotides according to the present invention show codon usage patterns which resemble those of E.coli and mammalian (e.g. human) genes. This is particularly advantageous where a sequence is to be used in vaccination of a mammal and in generation of significant amounts of the antigen protein in vitro using E.coli cells (e.g. for use in assays, such as immunoassays to judge the levels of expression in mammalian or human tissues).

As discussed above, the present invention includes expression vectors that comprise the nucleotide sequences of the invention. Such expression vectors are routinely constructed in the art of molecular biology and may for example involve the use of plasmid DNA and appropriate initiators, promoters, enhancers and other elements, such as for example polyadenylation signals which may be necessary, and which are positioned in the correct orientation, in order to allow for protein expression. Other suitable vectors would be apparent to persons skilled in the art. By way of further example in this regard we refer to Sambrook *et al.* Molecular Cloning: a Laboratory Manual. 2^{nd} Edition. CSH Laboratory Press. (1989).

Preferably, a polynucleotide of the invention, or for use in the invention in a vector, is operably linked to a control sequence which is capable of providing for the expression of the coding sequence by the host cell, i.e. the vector is an expression vector. The term "operably linked" refers to a juxtaposition wherein the components described are in a relationship permitting them to function in their intended manner. A regulatory sequence, such as a promoter, "operably linked" to a coding sequence is positioned in such a way that expression of the coding sequence is achieved under conditions compatible with the regulatory sequence.

The vectors may be, for example, plasmids, artificial chromosomes (e.g. BAC, PAC, YAC), virus or phage vectors provided with a origin of replication, optionally a promoter for the expression of the polynucleotide and optionally a regulator of the promoter. The vectors may contain one or more selectable marker genes, for example an ampicillin or kanamycin resistance gene in the case of a bacterial plasmid or a resistance gene for a fungal vector. Vectors may be used *in vitro,* for example for the production of DNA or RNA or used to transfect or transform a host cell, for example, a mammalian host cell e.g. for the production of protein encoded by the vector. The vectors may also be adapted to be used *in vivo,* for example in a method of DNA vaccination or of gene therapy.

Promoters and other expression regulation signals may be selected to be compatible with the host cell for which expression is designed. For example, mammalian promoters include the metallothionein promoter, which can be induced in response to heavy metals such as cadmium, and the β-actin promoter. Viral promoters such as the SV40 large T antigen promoter, human cytomegalovirus (CMV) immediate early (IE) promoter, rous sarcoma virus LTR promoter, adenovirus promoter, or a HPV promoter, particularly the HPV upstream regulatory region (URR) may also be used. All these promoters are well described and readily available in the art.

Examples of suitable viral vectors include herpes simplex viral vectors, vaccinia or alpha-virus vectors and retroviruses, including lentiviruses, adenoviruses and adeno-associated viruses. Gene transfer techniques using these viruses are known to those skilled in the art. Retrovirus vectors for example may be used to stably integrate the polynucleotide of the invention into the host genome, although such recombination is not preferred. Replication-defective adenovirus vectors by contrast remain episomal and therefore allow transient expression. Vectors capable of driving expression in insect cells (for example baculovirus vectors), in human cells or in bacteria may be employed in order to produce quantities of the HPV protein encoded by the polynucleotides of the present invention, for example for use as subunit vaccines or in immunoassays.

The polynucleotides according to the invention have utility in the production by expression of the encoded proteins, which expression may take place *in vitro, in vivo* or *ex vivo.* The nucleotides may therefore be involved in recombinant protein synthesis, for example to increase yields, or indeed may find use as therapeutic agents in their own right, utilised in DNA vaccination techniques. Where the polynucleotides of the present invention are used in the production of the encoded proteins *in vitro* or *ex vivo,* cells, for example in cell culture, will be modified to include the polynucleotide to be expressed. Such cells include transient, or preferably stable mammalian cell lines. Particular examples of cells which may be modified by insertion of vectors encoding for a polypeptide according to the invention include mammalian HEK293T, CHO, HeLa, 293 and COS cells. Preferably the cell line selected will be one which is not only stable, but also allows for mature glycosylation and cell surface expression of a polypeptide. Expression may be achieved in transformed oocytes. A polypeptide may be expressed from a polynucleotide of the present invention, in cells of a transgenic non-human animal, preferably a mouse. A transgenic non-human animal expressing a polypeptide from a polynucleotide of the invention is included within the scope of the invention.

Where the polynucleotides of the present invention find use as therapeutic agents, e.g. in DNA vaccination, the nucleic acid will be administered to the mammal e.g. human to be vaccinated. The nucleic acid, such as RNA or DNA, preferably DNA, is provided in the form of a vector, such as those described above, which may be expressed in the cells of the mammal. The polynucleotides may be administered by any available technique. For example, the nucleic acid may be introduced by needle injection, preferably intradermally, subcutaneously or intramuscularly. Alternatively, the nucleic acid may be delivered directly into the skin using a nucleic acid delivery device such as particle-mediated DNA delivery (PMDD). In this method, inert particles (such as gold beads) are coated with a nucleic acid, and are accelerated at speeds sufficient to enable them to penetrate a surface of a recipient (e.g. skin), for example by means of discharge under high pressure from a projecting device. (Particles coated with a nucleic acid molecule of the present invention are within the scope of the present invention, as are delivery devices loaded with such particles). The composition desirably comprises gold particles having an average diameter of 0.5-5µm, preferably about 2 µm. In preferred embodiments, the coated gold beads are loaded into tubing to serve as cartridges such that each cartridge contains 0.1-1 mg, preferably 0.5mg gold coated with 0.1-5 µg, preferably about 0.5 µg DNA/cartridge.

Suitable techniques for introducing the naked polynucleotide or vector into a patient include topical application with an appropriate vehicle. The nucleic acid may be administered topically to the skin, or to mucosal surfaces for example by intranasal, oral, intravaginal or intrarectal administration. The naked polynucleotide or vector may be present together with a pharmaceutically acceptable excipient, such as phosphate buffered saline (PBS). DNA uptake may be further facilitated by use of facilitating agents such as bupivacaine, either separately or included in the DNA formulation. Other methods of administering the nucleic acid directly to a recipient include ultrasound, electrical stimulation, electroporation and microseeding which is described in US-5,697,901.

Uptake of nucleic acid constructs may be enhanced by several known transfection techniques, for example those including the use of transfection agents. Examples of these agents includes cationic agents, for example, calcium phosphate and DEAE-Dextran and lipofectants, for example, lipofectam and transfectam. The dosage of the nucleic acid to be administered can be altered. Typically the nucleic acid is administered in an amount in the range of 1pg to 1 mg, preferably 1pg to 10µg nucleic acid for particle mediated gene delivery and 10µg to 1mg for other routes.

A nucleic acid sequence of the present invention may also be administered by means of specialised delivery vectors useful in gene therapy. Gene therapy approaches are discussed for example by Verme *et al,* Nature 1997, **389:**239-242. Both viral and non-viral vector systems can be used. Viral based systems include retroviral, lentiviral, adenoviral, adeno-associated viral, herpes viral, Canarypox and vaccinia-viral based systems. Non-viral based systems include direct administration of nucleic acids, microsphere encapsulation technology (poly(lactide-co-glycolide) and, liposome-based systems. Viral and non-viral delivery systems may be combined where it is desirable to provide booster injections after an initial vaccination, for example an initial "prime" DNA vaccination using a non-viral vector such as a plasmid followed by one or more "boost" vaccinations using a viral vector or non-viral based system.

A nucleic acid sequence of the present invention may also be administered by means of transformed cells. Such cells include cells harvested from a subject. The naked polynucleotide or vector of the present invention can be introduced into such cells *in vitro* and the transformed cells can later be returned to the subject. The polynucleotide of the invention may integrate into nucleic acid already present in a cell by homologous recombination events. A transformed cell may, if desired, be grown up *in vitro* and one or more of the resultant cells may be used in the present invention. Cells can be provided at an appropriate site in a patient by known surgical or microsurgical techniques (e.g. grafting, micro-injection, etc.)

Suitable cells include antigen-presenting cells (APCs), such as dendritic cells, macrophages, B cells, monocytes and other cells that may be engineered to be efficient APCs. Such cells may, but need not, be genetically modified to increase the capacity for presenting the antigen, to improve activation and/or maintenance of the T cell response, to have anti-tumour, e.g. anti-cervical carcinoma effects *per se* and/or to be immunologically compatible with the receiver (*i.e.,* matched HLA haplotype). APCs may generally be isolated from any of a variety of biological fluids and organs, including tumour and peri-tumoural tissues, and may be autologous, allogeneic, syngeneic or xenogeneic cells.

Certain preferred embodiments of the present invention use dendritic cells or progenitors thereof as antigen-presenting cells, either for transformation in vitro and return to the patient or as the in vivo target of nucleotides delivered in the vaccine, for example by particle mediated DNA delivery. Dendritic cells are highly potent APCs (*Banchereau* and Steinman, *Nature 392*:245-251, 1998) and have been shown to be effective as a physiological adjuvant for eliciting prophylactic or therapeutic antitumour immunity (see Timmerman and Levy, *Ann. Rev. Med. 50*:507-529, 1999). In general, dendritic cells may be identified based on their typical shape (stellate *in situ,* with marked cytoplasmic processes (dendrites) visible *in vitro*)*,* their ability to take up, process and present antigens with high efficiency and their ability to activate naïve T cell responses. Dendritic cells may, of course, be engineered to express specific cell-surface receptors or ligands that are not commonly found on dendritic cells *in vivo* or *ex vivo,* for example the antigen(s) encoded in the constructs of the invention, and such modified dendritic cells are contemplated by the present invention. As an alternative to dendritic cells, secreted vesicles antigen-loaded dendritic cells (called exosomes) may be used within a vaccine (see Zitvogel et al., *Nature Med. 4*:594-600, 1998).

Dendritic cells and progenitors may be obtained from peripheral blood, bone marrow, tumour-infiltrating cells, peritumoral tissues-infiltrating cells, lymph nodes, spleen, skin, umbilical cord blood or any other suitable tissue or fluid. For example, dendritic cells may be differentiated *ex vivo* by adding a combination of cytokines such as GM-CSF, IL-4, IL-13 and/or TNF to cultures of monocytes harvested from peripheral blood. Alternatively, CD34 positive cells harvested from peripheral blood, umbilical cord blood or bone marrow may be differentiated into dendritic cells by adding to the culture medium combinations of GM-CSF, IL-3, TNF, CD40 ligand, lipopolysaccharide LPS, flt3 ligand (a cytokine important in the generation of professional antigen presenting cells, particularly dentritic cells) and/or other compound(s) that induce differentiation, maturation and proliferation of dendritic cells.

APCs may generally be transfected with a polynucleotide encoding an antigenic HPV amino acid sequence, such as a codon-optimised polynucleotide as envisaged in the present invention. Such transfection may take place *ex vivo,* and a composition or vaccine comprising such transfected cells may then be used for therapeutic purposes, as described herein. Alternatively, a gene delivery vehicle that targets a dendritic or other antigen presenting cell may be administered to a patient, resulting in transfection that occurs *in vivo. In vivo* and ex *vivo* transfection of dendritic cells, for example, may generally be performed using any methods known in the art, such as those described in WO 97/24447, or the particle mediated approach described by Mahvi et al., *Immunology and cell Biology 75*:456-460, 1997.

Vaccines and pharmaceutical compositions may be presented in unit-dose or multi-dose containers, such as sealed ampoules or vials. Such containers are preferably hermetically sealed to preserve sterility of the formulation until use. In general, formulations may be stored as suspensions, solutions or emulsions in oily or aqueous vehicles. Alternatively, a vaccine or pharmaceutical composition may be stored in a freeze-dried condition requiring only the addition of a sterile liquid carrier immediately prior to use. Vaccines comprising nucleotide sequences intended for administration via particle mediated delivery may be presented as cartridges suitable for use with a compressed gas delivery instrument, in which case the cartridges may consist of hollow tubes the inner surface of which is coated with particles bearing the vaccine nucleotide sequence, optionally in the presence of other pharmaceutically acceptable ingredients.

The pharmaceutical compositions of the present invention may include adjuvant compounds, or other substances which may serve to modulate or increase the immune response induced by the protein which is encoded by the DNA. These may be encoded by the DNA, either separately from or as a fusion with the antigen, or may be included as non-DNA elements of the formulation. Examples of adjuvant-type substances which may be included in the formulations of the present invention include ubiquitin, lysosomal associated membrane protein (LAMP), hepatitis B virus core antigen, flt3-ligand and other cytokines such as IFN-γ and GMCSF.

Other suitable adjuvants are commercially available such as, for example, Freund's Incomplete Adjuvant and Complete Adjuvant (Difco Laboratories, Detroit, MI); Imiquimod (3M, St. Paul, MN); Resimiquimod (3M, St. Paul, MN); Merck Adjuvant 65 (Merck and Company, Inc., Rahway, NJ); AS-2 (SmithKline Beecham, Philadelphia, PA); aluminium salts such as aluminium hydroxide gel (alum) or aluminium phosphate; salts of calcium, iron or zinc; an insoluble suspension of acylated tyrosine; acylated sugars; cationically or anionically derivatized polysaccharides; polyphosphazenes; biodegradable microspheres; monophosphoryl lipid A and quil A. Cytokines, such as GM-CSF or interleukin-2, -7, or -12, may also be used as adjuvants.

In the formulations of the invention it is preferred that the adjuvant composition induces an immune response predominantly of the Th1 type. Thus the adjuvant may serve to modulate the immune response generated in response to the DNA-encoded antigens from a predominantly Th2 to a predominantly Th1 type response. High levels of Th1-type cytokines (*e.g.*, IFN-, TNF, IL-2 and IL-12) tend to favour the induction of cell mediated immune responses to an administered antigen. Within a preferred embodiment, in which a response is predominantly Th1-type, the level of Th1-type cytokines will increase to a greater extent than the level of Th2-type cytokines. The levels of these cytokines may be readily assessed using standard assays. For a review of the families of cytokines, see Mosmann and Coffman, *Ann. Rev. Immunol. 7*:145-173, 1989.

Accordingly, suitable adjuvants for use in eliciting a predominantly Th1-type response include, for example, a combination of monophosphoryl lipid A, preferably 3-de-O-acylated monophosphoryl lipid A (3D-MPL) together with an aluminium salt. Other known adjuvants which preferentially induce a TH1 type immune response include CpG containing oligonucleotides. The oligonucleotides are characterised in that the CpG dinucleotide is unmethylated. Such oligonucleotides are well known and are described in, for example WO96/02555. Immunostimulatory DNA sequences are also described, for example, by Sato et al., *Science 273*:352, 1996. CpG-containing oligonucleotides may be encoded separately from the papilloma antigen(s) in the same or a different polynucleotide construct, or may be immediately adjacent thereto, e.g. as a fusion therewith. Alternatively the CpG-containing oligonucleotides may be administered separately i.e. not as part of the composition which includes the encoded antigen. CpG oligonucleotides may be used alone or in combination with other adjuvants. For example, an enhanced system involves the combination of a CpG-containing oligonucleotide and a saponin derivative particularly the combination of CpG and QS21 as disclosed in WO 00/09159 and WO 00/62800. Preferably the formulation additionally comprises an oil in water emulsion and/or tocopherol.

Another preferred adjuvant is a saponin, preferably QS21 (Aquila Biopharmaceuticals Inc., Framingham, MA), which may be used alone or in combination with other adjuvants. For example, an enhanced system involves the combination of a monophosphoryl lipid A and saponin derivative, such as the combination of QS21 and 3D-MPL as described in WO 94/00153, or a less reactogenic composition where the QS21 is quenched with cholesterol, as described in WO 96/33739. Other preferred formulations comprise an oil-in-water emulsion and tocopherol. A particularly potent adjuvant formulation involving QS21, 3D-MPL and tocopherol in an oil-in-water emulsion is described in WO 95/17210.

Other preferred adjuvants include Montanide ISA 720 (Seppic, France), SAF (Chiron, California, United States), ISCOMS (CSL), MF-59 (Chiron), Detox (Ribi, Hamilton, MT), RC-529 (Corixa, Hamilton, MT) and other aminoalkyl glucosaminide 4-phosphates (AGPs).

Other preferred adjuvants include adjuvant molecules of the general formula (I)

Formula (I): HO(CH2CH2O)n-A-R

wherein, n is 1-50, A is a bond or-C(O)-, R is C1-50 alkyl or Phenyl C1-50 alkyl.

One embodiment of the present invention consists of a formulation comprising a polyoxyethylene ether of general formula (I), wherein *n* is between 1 and 50, preferably 4-24, most preferably 9; the R component is C1-50, preferably C4-C20 alkyl and most preferably C12 alkyl, and *A* is a bond. The concentration of the polyoxyethylene ethers should be in the range 0.1-20%, preferably from 0.1-10%, and most preferably in the range 0.1-1%. Preferred polyoxyethylene ethers are selected from the following group: polyoxyethylene-9-lauryl ether, polyoxyethylene-9-steoryl ether, polyoxyethylene-8-steoryl ether, polyoxyethylene-4-lauryl ether, polyoxyethylene-35-lauryl ether, and polyoxyethylene-23-lauryl ether. Polyoxyethylene ethers such as polyoxyethylene lauryl ether are described in the Merck index (12th edition: entry 7717). These adjuvant molecules are described in WO 99/52549. The polyoxyethylene ether according to the general formula (I) above may, if desired, be combined with another adjuvant. For example, a preferred adjuvant combination is preferably with CpG as described in the pending UK patent application GB 9820956.2.

Where the vaccine includes an adjuvant, the vaccine formulation may be administered in two parts. For example, the part of the formulation containing the nucleotide construct which encodes the antigen may be administered first, e.g. by subcutaneous or intramuscular injection, or by intradermal particle-mediated delivery, then the part of the formulation containing the adjuvant may be administered subsequently, either immediately or after a suitable time period which will be apparent to the physician skilled in the vaccines arts. Under these circumstances the adjuvant may be administered by the same route as the antigenic formulation or by an alternate route. In other embodiments the adjuvant part of the formulation will be administered before the antigenic part. In one embodiment, the adjuvant is administered as a topical formulation, applied to the skin at the site of particle mediated delivery of the nucleotide sequences which encode the antigen(s), either before or after the particle mediated delivery thereof.

The following Examples serve to further illustrate the invention, with reference to the accompanying drawings, in which:
- Figure 1: shows the prototype wild-type amino acid sequences of E1 from HPV types 11, 6a and 6b, derived from Genbank;
- Figure 2: shows the prototype wild-type amino acid sequence of HPV6b E1 from Fig. 1 (6b-e1) aligned with the HPV6b E1 amino acid sequence including point mutations to remove biological activity (6b-e1 mut);
- Figure 3: shows the prototype wild-type amino acid sequences of E2 from HPV types 11, 6a and 6b, derived from Genbank;
- Figure 4a: shows the prototype wild-type amino acid sequence of HPV11 E2 from Fig. 3 (Hpv-11e2-wt) aligned with the HPV11 E2 amino acid sequence including a point mutation to remove biological activity (Hpv-11 e2-mut) and with the amino acid sequence encoded by the nucleotide sequence of Fig. 6 (Hpv-11e2-comut);
- Figure 4b: shows the prototype wild-type amino acid sequence of HPV6b E2 from Fig. 3 (Hpv-6be2-wt) aligned with the HPV6b E2 amino acid sequence including a point mutation to remove biological activity (Hpv-6be2-mut);
- Figure 5: shows a nucleotide sequence, having a codon usage pattern resembling that of a highly expressed human gene, encoding the mutated amino acid sequence of HPV6b E1 from Fig. 2;
- Figure 6: shows a nucleotide sequence, having a codon usage pattern resembling that of a highly expressed human gene, encoding the mutated amino acid sequence of HPV11 E2 from Fig. 4;
- Figure 7: shows DNA vector p7313-PLc;
- Figure 8: shows cell lysate samples from Example 4 run on an acrylamide gel and stained to show antibody binding to expressed E1 protein;
- Figure 9: shows cellular responses to antigen challenge after immunisation of mice with a polynucleotide according to the invention (Example 6); and
- Figure 10: shows cell lysate samples from Example 7 run on an acrylamide gel and stained to show antibody binding to expressed E2 protein.

### Example 1 - Codon Optimisation of HPV6bE1

The wild-type prototype amino acid sequence of HPV6b E1, obtained from Genbank, is set out in Fig.1 (bottom sequence). This figure shows the high level of homology for this protein between the HPV virus prototype sequences of types 11, 6a and 6b. Similarly, Figure 3 sets out the wild-type prototype amino acid sequences for the E2 protein of HPV11, 6a and 6b. It is expected that an immune therapy (therapeutic vaccine) using HPV6b sequences will cross-react to provide a prophylactic or therapeutic immune response against all three viral types.

The codon usage of the HPV6b E1 sequence was compared to that of highly expressed human and E.coli genes and found to have a low codon usage coefficient for both species. Simply using the most abundant codon for each amino acid residue would also result in a skewed codon usage pattern, as no organism uses exclusively its most preferred codon for a given amino acid. Consequently, the codons were assigned using a statistical method to give a synthetic gene having a codon frequency closer to that found naturally in highly expressed E.coli and human genes.

The codons in the synthetic gene were assigned using a Visual Basic program called Calcgene, written by R. S. Hale and G Thompson (Protein Expression and Purification Vol. 12 pp.185-188 (1998)). For each amino acid residue in the original sequence, a codon was assigned based on the probability of it appearing in highly expressed E.coli genes. Details of the program, which works under Microsoft Windows 3.1, can be obtained from the authors. Because the program applies a statistical method to assign codons to the synthetic gene, not all resulting codons are the most frequently used in the target organism. Rather, the proportion of frequently and infrequently used codons of the target organism is reflected in the synthetic sequence by assigning codons in the correct proportions. However, as there is no hard-and-fast rule assigning a particular codon to a particular position in the sequence, each time it is run the program will produce a different synthetic gene - although each will have the same codon usage pattern and each will encode the same amino acid sequence. If the program is run several times for a given amino acid sequence and a given target organism, several different nucleotide sequences will be produced which may differ in the number, type and position of restriction sites, intron splice signals etc., some of which may be undesirable. The skilled artisan will be able to select an appropriate sequence for use in expression of the polypeptide on the basis of these features.

Furthermore, since the codons are randomly assigned on a statistical basis, it is possible (although perhaps unlikely) that two or more codons which are relatively rarely used in the target organism might be clustered in close proximity. It is believed that such clusters may upset the machinery of translation and result in particularly low expression rates, so the algorithm for choosing the codons in the optimized gene excluded any codons with an RSCU value of less than 0.2 for highly expressed genes in order to prevent any rare codon clusters being fortuitously selected. The distribution of the remaining codons was then allocated according to the frequencies for highly expressed E.coli genes to give an overall distribution within the synthetic gene that resembled that of the E.coli genes (coefficient = 0.85) and also that of highly expressed human genes (coefficient = 0.50). A similar process was used to obtain a codon optimised nucleotide sequence for HPV11 E2, except that Syngene (Peter Ertl, unpublished), an updated version of the Calcgene program, allowing exclusion of rare codons to be optional, was used to allocate codons according to the codon frequency pattern of highly expressed human genes. Unlike the codon assignment for E1, rare codons were not excluded. At the same time, an alteration was made to one of the oligonucleotides to encode a K111A amino acid change, as described below.

Mutations were introduced into the codon optimised E1 and E2 genes to give rise to point mutations in the E1 (K83G, R84G and G483D) and E2 (K111A) amino acid sequences. The amino acid sequences of the mutated genes are shown (aligned with the wild-type prototype sequence) in Figs. 2 (HPV6bE1) and 4 (HPV6bE2 and HPV11E2). The codon optimised and mutated nucleotide sequence for HPV6b E1 is shown in Fig. 5. The codon optimised and mutated nucleotide sequence for HPV11 E2 is shown in Fig. 6. In Figure 4, the amino acid sequence of the polypeptide obtained by expression of the codon optimised and mutated HPV11E2 gene is also given, in alignment with the prototype wild-type and the mutated wild-type sequences, to show that codon optimisation of the nucleotide sequence does not alter the amino acid sequence encoded (which is identical to the mutated wild-type sequence).

### Example 2 - Construction of the Codon Optimised HPV6b E1 Polynucleotide Sequence

### Gene Design:

Using the optimisation software discussed above, overlapping 40mer oligonucleotides were calculated from the optimised sequence. The terminal oligonucleotides containing the restriction sites were 60mers. The oligonucleotides were ordered from Life Technologies Ltd at 50nmole concentration, deprotected and non-phosphorylated.

### Oligonucleotide assembly:

Each oligonucleotide was dissolved in double distilled water to a final concentration of 100 micromolar (µM) and an equal mixture was prepared of all 96 oligonucleotides at 100 µM. The synthesis was set up as follows using Pwo polymerase from Roche Boehringer (Cat No. 1 644 955).

| | |
|---|---|
| Double distilled water | 86µl |
| Pwo 10X buffer | 10µl |
| dNTP mix | 1µl (equal mix of 100mM dNTPs) |
| oligo mix | 1µl (equal mix of 100µM oligos) |
| Pwo polymerase | 2µl. |

A Polymerase Chain Reaction (PCR) was carried out on the above reaction mix on a Trio Thermoblock (Biometra)using the following conditions:

| | |
|---|---|
| 1. | 40°C 2 min |
| 2. | 72°C 10 sec |
| 3. | 94°C 15 sec |
| 4. | 40°C 30 sec |
| 5. | 72°C 20 sec + 2secs per cycle |
| 6. | 4°C ∞ |

Cycle repeated 25 times between steps 3 and 5.

After completion of 25 cycles, a 10µl aliquot was removed from each tube and run on a 0.8% Tris Acetate (TAE) agarose gel and observed under long wave UV light. The expected size of the synthesised E1 DNA should be approx. 2kb.

### Gene Recovery:

The synthetic gene was recovered by PCR using polymerase using the two terminal oligos which contained a Not 1 restriction site on the N terminal of the synthetic oligo and a Bam H1 site at the C terminal synthetic oligo.

| | |
|---|---|
| Double distilled water | 65µl |
| Pwo 10X buffer | 10µl |
| dNTP mix | 1µl (equal mix of 100mM dNTPs) |
| assembly mix | 20µl (from previous PCR) |
| N terminal oligo | 1µl (100µM) |
| C terminal oligo | 1µl (100µM) |
| Pwo polymerase | 2µl. |

| | |
|---|---|
| 1. | 94°C 45 sec |
| 2. | 72°C 2 min + 1 min per 500bp |
| 3. | 72°C 10 min |
| 4. | 4°C ∞ |

Cycle repeated 25 times between steps 2 and 1.

The PCR product was then purified using a QlAquick PCR purification kit (Qiagen Cat No. 28104) before the DNA was resuspended in a total of 50µl of kit elution buffer. A 10µl aliquot was digested with Not 1 and BamH1 restriction enzymes (from Life Technologies Ltd, 3 Fountain Drive, Inchinnan Business Park, Paisley, Scotland) for 2 hours at 37°C. This digest was gel purified on 0.8% TAE agarose gel and the 2kb DNA product excised and extracted using a QlAquick Gel extraction Kit (Qiagen Cat No. 28704). The final digested pure DNA fragment was eluted in a total of 50µl of kit elution buffer.

This PCR fragment was cloned into vector p7313PLc (Fig. 7), (Powderject Vaccines Inc., see further details below) and transformed into competent JM109 cells (Promega cat no: P9751). Plasmid DNA from selected clones were restriction enzyme checked by digestion with Nco1-BamH1 and Nco1-EcoR1. Five correct clones with 2kb fragment inserts were selected and the insert DNA's sequenced. One clone with an insert containing just three point mutations was selected for further use. The three point mutations were corrected by ligation swap with homologous small fragments from other clones.

The corrected clone was re-checked by restriction enzyme digestion and the insert DNA fully sequenced. This cloned was designated p6bE1c/o. At the same time and for comparative expression and immunisation studies the wild type HPV-6b E1 gene, and the wild type HPV-11 E1 gene were PCR amplified from genomic clones of HPV-6b, (EMBO J. 2 (12) 2314-2318 1983) and HPV-11 (Virology 151, 124-130 1986), and the respective fragments cloned into Not 1 - BamH1 digested vector p7313PLc. These clones were designated p6bE1w/t and p11E1w/t respectively.

The E1 genes in clones p6bE1c/o and p6bE1w/t were further mutated to introduce amino acid changes K83G, R84G and G438D. Sequenced matched 3' and 5' oligonucleotide primers with nucleotide substitutions designed to introduce the required mutations were used in PCR reactions with other kit reagents by methods described in QuickChange Site-Directed Mutagenesis Kit (Stratagene Cat No. 200518). Mutated p6bE1c/o and p6bE1w/t clones were designated p6bE1c/o mut and, p6bE1w/t mut respectively.

### Example 3 - Construction of the Codon Optimised HPV11 E2 Polynucleotide Sequence

The design, assembly and recovery of the E2 codon optimised gene was as described above for E1, but the overlapping oligonucleotides were 60 nucleotides in length rather than 40, with an 18nt overlap. Unlike in the E1 procedure a clone containing the K111A amino acid mutation was generated at the same time as the codon optimised E2 gene clone by substituting the two appropriate wild type sequence oligonucleotides with two 60mer oligonucleotides which together comprise the nucleotide substitution required to generate the K111A change.

### Composition of plasmid p7313-PLc

The plasmid was constructed by replacing the beta-lactamase gene containing Eam11051 - Pst1 fragment of pUC19 (available from Amersham Pharmacia Biotech UK Ltd., Amersham Place, Little Chalfont, Bucks, HP7 9NA) with an EcoRI fragment of pUC4K (Amersham-Pharmacia) containing the Kanamycin resistance gene, following blunt ending of both fragments using T4 DNA polymerase. The human Cytomegalovirus IE1 promoter /enhancer, Intron A, was derived from plasmid JW4303 obtained from Dr Harriet Robinson, University of Massachusetts, and inserted into the Sal1 site of pUC19 as a Xhol -Sal1 fragment, incorporating the bovine growth hormone polyadenylation signal. Deletion of the 5' Sall-Banl fragment from the promoter generated the minimal promoter used in the vector (WO00/23592 - Powderject Vaccines Inc.). HBV Surface antigen 3'UTR was derived from Hepatitis B Virus, serotype adw, in the vector pAM6 (Moriarty et al., Proc.Natl.Acad.Sci. USA, 78, 2606-2610, 1981). pAM6 (pBR322 based vector) was obtained from the American Type Culture Collection, catalogue number ATCC 45020. The 3'UTR was inserted 5' to the polyadenylation signal as a 1.4kb BamHl fragment, blunt ended for insertion to remove the BamHl sites. In a series of steps (including digestion with *Bgl* II, Klenow polymerase treatment, digestion with *Bst*X I, digestion with Nco I, treatment with mung bean nuclease to remove overhang and further digestion with *Bst*X I), modifications were made to the region between the 3'untranslated enhancer region of the HBV S gene and bGHpA signal to remove all open reading frames of greater than 5 codons between the X gene promoter and the bGHpA signal. This resulted in deletion of sequence encoding the translatable portion of the X protein (9 amino acids) and the X gene start codon. However, the weak enhancer/promoter region of the X gene was retained because this region was found to enhance expression of HBsAg from the CMV promoter. The bovine growth hormone polyadenylation signal was substituted with the rabbit beta globin polyadenylation signal. The 5'non-coding and coding sequences of the S antigen were excised and replaced with an oligonucleotide linker to provide multiple cloning sites as shown to produce plasmid p7313-PL.

Hind -- - Not I - -- -EcoRV --NdeI-- --BamHI
AGCTTGCGGCCGCTAGCGATATCGGTACCATATGTCGACGGATCC . . . .
. . . . ACGCCGGCGATCGCTATAGCCATGGTCTACAGCTGCCTAGGCCGG
--NheI-- --KpnI-- -- SalI -- ΔNotI

The ColE1 cer sequence was obtained from a subclone from plasmid pDAH212 from David Hodgeson (Warwick University) and amplified by PCR using primers to place EcoRI restriction sites at the ends of the sequence. The cer sequence was then inserted into the EcoRI site of p7313-PL to produce plasmid p7313-PLc (Fig. 7). The sequence of the amplified cer was verified against the Genbank entry M11411.

### Example 4 - Expression of E1 in mammalian 293T cells

Mammalian 293T cells were grown at log phase at a final concentration of 2 X10⁵ cells per 6 well Corning Costar^{™} (Corning Science Products, 10 The ValleyCentre, Gordon Road, High Wycombe, Bucks, UK) tissue culture plate overnight at 37°C in 5%CO₂. The following transfection mix was prepared and complexed for 25 minutes:
2µg of plasmid DNA (vector, p6bE1c/o, p6bE1w/t) in 16µl sterile double distilled water
plus:

| | |
|---|---|
| OPTI-mem^{™} (Gibco BRL, Paisley, Scotland) | 8µl |
| Lipofectamine^{™} (GibcoBRL) | 6µl. |

Each cell monolayer was washed carefully twice with OPTI-mem^{™}. 800µl of OPTI-mem^{™} was added to each well. 200µl of OPTI-mem^{™} was added to each transfection mix, mixed and added gently to a cell monolayer. The plate was incubated for 5 hours at 37°C in 5% CO₂ after which the transfection mix and OPTI-mem^{™} were discarded. The cell monolayers were washed gently with cell growth medium twice and finally transfected cells were incubated for 24 hours in Dulbecco's Modified Eagle Medium containing 10% foetal calf serum and 29.2mg/ml of L-glutamine at 37°C in 5% CO₂. The cells were scraped off into microtubes, washed twice with PBS, spun down and the cell pellet was resuspended in SDS Page Laemmli dye. The cell pellets were boiled and loaded onto a 10% SDS Page gel and electrophoresed in 1X Tris Glycine SDS buffer. After electrophoresis, the gel was blotted onto Nitrocellulose membrane (Amersham) and Western Blotted. The nitrocellulose membrane was blocked with 5% Marvel^{™} (Premier Beverages, Knighton, Adbaston, Stafford, UK) in PBS for 30 min at room temperature and washed twice with PBS and 0.1% Tween 20. A polyclonal antibody raised against the C terminal protein sequence of HPV6bE1 (protein sequence: CSSSLDIQDSEDEEDGSNSQAFR) in rabbits, was diluted in 5% Marvel^{™} in PBS and added to the nitrocellulose membrane. This was incubated at room temperature for 1 hour with gentle agitation. The diluted antibody was removed and the membrane washed three times with PBS and 0.1% Tween 20. A secondary conjugate, Swine anti-rabbit horseradish peroxidase (HRP) (DAKO), was diluted 1:20000 in PBS and 0.1% Tween 20. This was added to the washed membrane and incubated with gentle agitation at room temperature for 1 hour. The membrane was then washed thoroughly with PBS and 0.1% Tween20. A Chemiluminescent HRP kit (Amersham) was used to detect the transferred proteins on the membrane.

### Results:

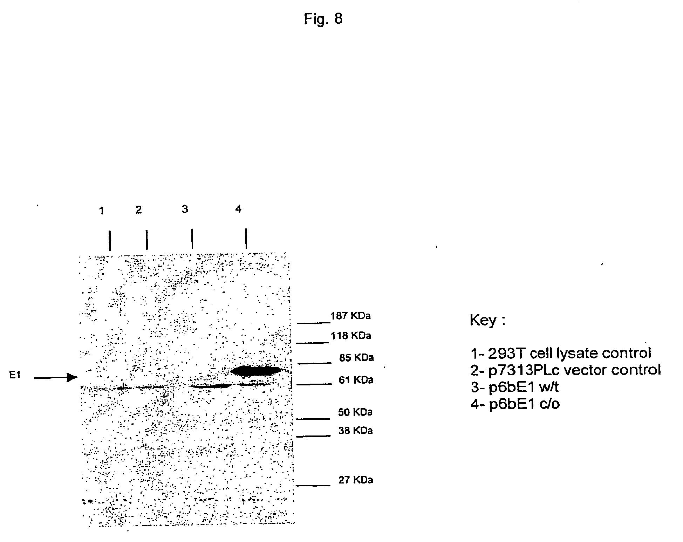

The predicted size of a translated protein for E1 is 68kDa - 72kDa. The results (Fig. 8) show a correct protein size expressed by p7313-PLc containing the codon optimised HPV6bE1 (lane 4). Vector containing wild-type E1 is in lane 3, which shows that there was no detectable expression of E1 in human cells from the wild-type nucleotide sequence. Similarly no E1 is detected in lane 2 (empty vector) and lane 1 (untransfected cells). The approx. 60kD band in lanes 1-4 is an unidentified cellular protein which cross-reacts with the anti-E1 antibody. The band is of roughly constant intensity across the lanes, showing that the loading of the samples was consistent.

### Example 5 - Construction of recombinant Vaccinia Virus Expressing HPV E1 and HPV E2 proteins.

Vaccinia virus expressing HPV-6b E1 protein was generated at Glaxo Wellcome, Stevenage, UK. Vaccinia virus expressing HPV-11 E1 and HPV-11 E2 were a kind gift from Jeff Engler, University of Alabama at Birmingham, US.

Briefly, the E1 gene from p6bE1w/t was cloned into Vaccinia virus vector pTM3 and then the restriction enzyme checked and DNA sequenced recombinant vector used to transfected HTk⁻ cells. Recombinant Vaccinia virus was isolated and plaque purified. E1 protein expression was checked by Western blotting using peptide antisera after infection of permissive cells with both the recombinant virus expressing HPV-6b E1 and, a second Vaccinia virus (vTF7-3) expressing bacteriophage T7 RNA polymerase. Co-infection of cells with Vaccinia virus vFT7-3 is also necessary in order to direct expression of E1 and E2 protein from the HPV-11 E1 and E2 recombinant Vaccinia viruses. Vaccinia virus strain WR was used in negative control experiments. Vector pTM3 and virus vTF7-3 were from National Institute of Health, Maryland, US.

### Example 6 - Immunology - detection of cellular responses to HPV antigens

All reagents were obtained from Gibco BRL, Paisley, Scotland or Sigma, Poole, Dorset unless otherwise stated.

### A. Immunisation protocol.

Female C57BU6 mice were immunised with 1.0-2.0 µg DNA (either p6bE1c/o, p6bE1w/t, p6bE1c/o mut, p6bE1w/t mut or empty plasmid p7313PLc) by PMDD and boosted with an identical dose 14 days later. Animals were sacrificed by cervical dislocation and spleens removed for investigation of the cellular responses to HPV antigens.

### B. Preparation of single cell suspension of splenocytes.

Spleens were "mashed" between ground glass slides (BDH), red blood cells lysed (155 mM NH₄Cl, 10 mM KHCO₃, 0.1 mM EDTA) and cells resuspended in complete RPMI. (RPMI-1640 medium supplemented with 10% foetal calf serum (FCS), 2mM glutamine, 100 units/ml penicillin, 100 µg/ml streptomycin and 5 x 10⁻⁵ M 2-mercaptoethanol).

### C. Infection of MC57 target cells.

Immunodominant epitopes derived from HPV antigens remain undefined therefore the detection of antigen specific responses *in vitro* relies on natural processing of whole antigen generated within target cells that have been transfected with cDNA encoding the whole protein(s). MC57 cells (K^{b} positive) were infected with recombinant vaccinia virus expressing HPV-6b E1, HPV-11 E2 or HPV-11 E1 using a multiplicity of infection of 5 for 1 hour at 37°C. Excess virus was washed off and cells resuspended in complete RPMI containing 50 ng/ml recombinant human IL-2 (Glaxo Wellcome, Geneva).

### D. ELISPOT.

ELISPOT plates (96 well, Millipore MAIP S 45 1 0) were coated with rat anti-mouse IFN gamma (Pharmingen 18181D) at 15 µg/ml in PBS overnight (4°C) prior to the addition of 4 x 10e5 splenocytes obtained from experimental groups. Antigen was presented by the addition of 1 x 10e4 recombinant vaccinia infected MC57 cells. Wild-type vaccinia strain WR was used as a negative control. The assay was incubated overnight at 37°C(5% CO₂).

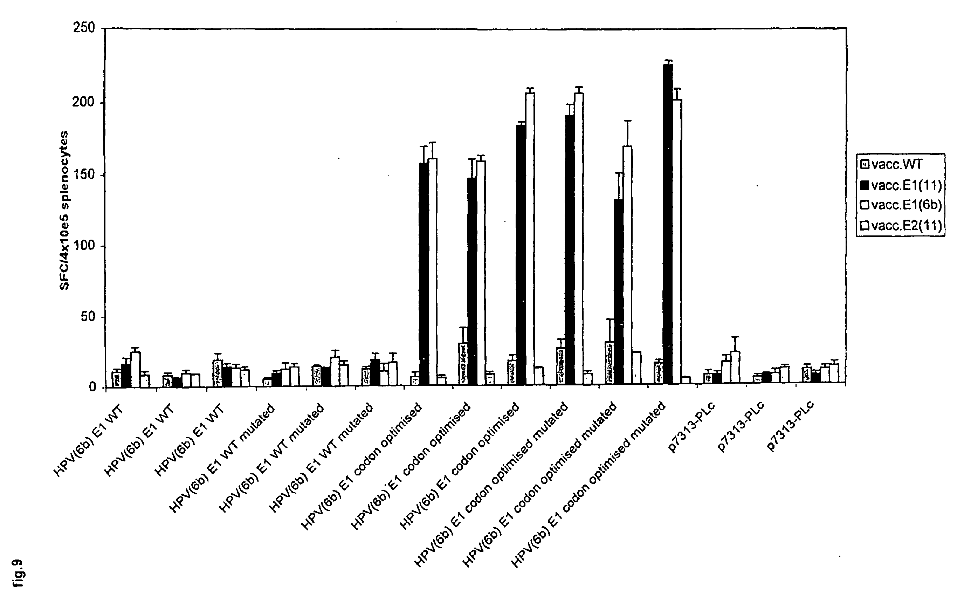

On day 2 of the assay, spot forming cells were detected using biotinylated rat anti-mouse IFN gamma (Pharmingen 18112D) at 1 µg /ml followed by streptavidin alkaline phosphatase conjugate (TCS biologicals SA 1008) at 1/1000 dilution in PBS. This was visualised using an alkaline phosphatase substrate kit (Biorad 170-6432) and quantified by image analysis. The results are shown in Fig. 9.

As can be seen from Fig. 9, a strong cellular response was seen from all three mice vaccinated with plasmid encoding HPV-6b codon optimised E1 sequence, when challenged with E1 carried in the vaccinia vector (vacc.E1(11) or vacc.E1(6b)). No response was seen when these mice were challenged with wild-type vaccinia (vacc.WT), or with vaccinia expressing HPV-11 E2 (vacc.E2(11)). By contrast, vaccination of mice with plasmid encoding the wild type E1 sequence does not result in a T-cell response. Splenocytes from these mice do not react to challenge by vaccinia carrying the E1 gene, nor to any other challenge (data not shown). Mutation of the E1 gene does not alter the cellular response in mice. Also, since mice immunised with p6bE1 c/o and p6bE1 c/o mut raised strong cellular immune responses against target cells infected with a Vaccinia virus expressing E1 protein from HPV-11, we can assume that there is a high level of immunlogic cross reactivity between HPV-6b E1 and HPV-11 E1. Mice vaccinated with empty p7313-PLc vector showed no response to any challenge.

### Example 7 - Expression of HPV 6b E2 in mammalian 293T cells

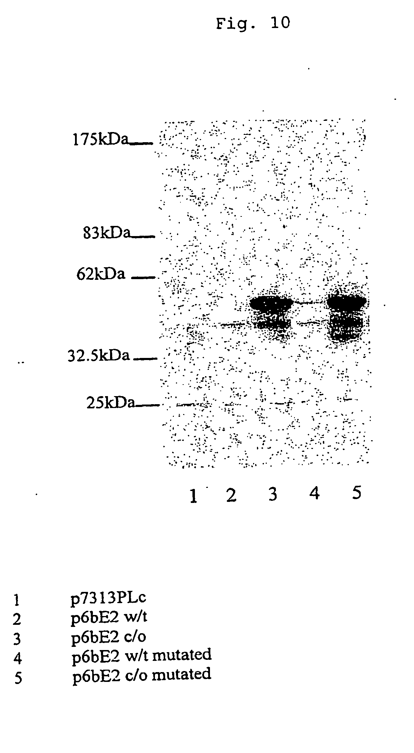

293T cell monolayers (80% confluent) in 24 well plates were transfected with 1µg of each plasmid (p6bw/t, p6bc/o, p6bw/t mut, p6bc/o mut) using 2.5µl of Lipofectamine 2000 (Life Technologies) per transfection following the standard protocol (see Example 4 above). 24hrs after transfection the cells were harvested, rinsed in phosphate buffered saline and examined by SDS-PAGE and Western blot using an anti-E2 peptide antiserum (#1100) raised against HPV-6b N-terminal amino acid sequence MEAIAKRLDACQEQLLELYEEC (Fig. 10).

### Results

The results show a major protein band of the expected size (40kd - 45 kD) in track 3 (codon optimised E2) and track 5 (codon optimised and mutated E2). A minor band of the same size also appears in track 4 indicating some very low level expression from the mutated wild type E2 plasmid, but not in track 1 (negative control), or in track 2 (wild type E2 protein). A cross reacting protein band of ~25kd appears in all tracks indicating equal loading of protein lysates. Mutation of E2 does not appear to compromise E2 protein expression which is significant improved by codon-optimisation.

## Claims

1. A polynucleotide sequence which encodes a human papilloma virus amino acid sequence of an HPV Early antigen or fragment thereof said polynucleotide being capable of raising an immune response when administered in vivo **characterised in that** said polynucleotide has a codon usage coefficient of greater than 0.5 but less than 1.0 for a highly expressed human gene.

2. A polynucleotide sequence according to any one of claim 1 which is a DNA sequence.

3. A polynucleotide sequence according to claim 1 or 2 which encodes a HPV polypeptide of a HPV type or sub-type associated with cervical cancer, benign cutaneous warts or genital warts.

4. A polynucleotide sequence according to claim 3 which encodes a HPV polypeptide of one of types 1-4, 6, 7, 10, 11, 16, 18, 26-29, 31, 33, 35, 39, 49, 51, 52, 56, 58, 59 and 68.

5. A polynucleotide sequence according 4 which encodes a HPV polypeptide of an HPV type or sub-type which is associated with cervical cancer or genital warts.

6. A polynucleotide sequence according to claim 5 which encodes a HPV polypeptide of one of types 6, 11, 16, 18, 33 or 45, or a fusion of two or more polypeptides of one or more of HPV virus types 6, 11, 16, 18, 33 or 45.

7. A polynucleotide sequence according to claim 5 which encodes a HPV polypeptide of a HPV type or sub-type selected from HPV 11, 6a or 6b.

8. A polynucleotide sequence according to any preceding claim which encodes a mutated HPV E1 polypeptide having the following mutations K83G, R84G and G483D.

9. A polynucleotide sequence according to any claims of 1 to 7 which encodes a mutated HPV E2 polypeptide comprising an KIIIA mutation.

10. A polynucleotide sequence according to any preceding claim in which the encoded HPV polypeptide comprises the whole or a part of a HPV early gene product.

11. A polynucleotide sequence according to claim 9 in which the encoded HPV polypeptide comprises the whole or a part of E1 or E2, or a fusion of the whole or a part of E1 or E2 with another HPV polypeptide.

12. A polynucleotide sequence according to any preceding claim having a codon usage coefficient for E.coli genes of greater than 0.6.

13. A polynucleotide sequence as set out in (Seq ID No 10, Fig. 5), or a fragment or analogue thereof which retains the codon usage thereof.

14. A polynucleotide sequence as set out in (Seq ID No 11, Fig. 6), or a fragment or analogue thereof which retains the codon usage thereof.

15. An expression vector comprising a polynucleotide sequence according to any preceding claim operably linked to a control sequence which is capable of providing for the expression of the polynulceotide sequence by a host cell.

16. An expression vector according to claim 15 which is capable of directing the expression of the polynulceotide sequence in bacterial insect or mammalian cells.

17. An expression vector according to claim 15 or claim 16 wherein the vetor backbone is p7313PLc as shown in figure 7.

18. A host cell comprising a polynucleotide sequence according to any one of claims 1-14.

19. A host cell comprising an expression vector according to any one of claims 15-17.

20. A host cell according to claim 18 or claim 19 which is bacterial, mammalian, or insect cell.

21. A pharmaceutical composition comprising a polynucleotide sequence according to any one of claims 1-14.

22. A pharmaceutical composition comprising a vector according to any one of claims 15-17.

23. A pharmaceutical composition according to claim 21 or claim 22 comprising a plurality of particles, preferably gold particles, coated with DNA.

24. A pharmaceutical composition according to claim 22 comprising of a pharmaceutically acceptable excipient and the DNA vector.

25. A pharmaceutical composition according to any one of claims 21-24 further comprising an adjuvant,

26. A pharmaceutical composition according to claim 25 in which the adjuvant is encoded as a fusion with the HPV polypeptide encoded by the polynucleoide.

27. Use of a polynucleotide according to any one of claims 1-14 in the manufacture of a medicament for the treatment or prophylaxis of an HPV infection.

28. Use of a vector according to any one of claims 15-17 in the manufacture of a medicament for the treatment or prophylaxis of a HPV infection.

29. Use according to any one of claims 27 and 28 in which the HPV infection is an infection of one or more of HPV type 6, 11, 16 or 18.

30. The use of a polynucleotide according to any one of claims 1-14, a vector according to any one of claims 15-17 in the manufacture of a medicament for the treatment or prophylaxis of cutaneous (skin) warts, genital warts, atypical squamous cells of undetermined significance (ASCUS), cervical dysplasia, cervical intraepithelial neoplasia (CIN) or cervical cancer.

## Patentansprüche

1. Polynucleotidsequenz, die eine Aminosäuresequenz eines menschlichen Papillomavirus eines HPV-"Early"-Antigens oder eines Fragments davon codiert, wobei das Polynucleotid in der Lage ist, eine Immunantwort auszulösen, wenn es in vivo verabreicht wird, **dadurch gekennzeichnet, dass** dieses Polynucleotid einen Codon-Verwendungskoeffizienten von mehr als 0,5, aber weniger als 1,0, eines stark exprimierten menschlichen Gens hat.

2. Polynucleotidsequenz gemäß Anspruch 1, welche eine DNA-Sequenz ist.

3. Polynucleotidsequenz gemäß Anspruch 1 oder 2, welche ein HPV-Polypeptid oder ein HPV-Typ oder -Subtyp codiert, der mit Gebärmutterhalskrebs, benignen Hautwarzen oder Genitalwarzen assoziiert ist.

4. Polynucleotidsequenz gemäß Anspruch 3, welche ein HPV-Polypeptid eines der Typen 1-4, 6, 7, 10, 11, 16, 18, 26-29, 31, 33, 35, 39, 49, 51, 52, 56, 58, 59 und 68 codiert.

5. Polynucleotidsequenz gemäß Anspruch 4, welche ein HPV-Polypeptid codiert, oder einen HPV-Typ oder -Subtyp, der mit Gebärmutterhalskrebs oder Genitalwarzen assoziiert ist.

6. Polynucleotidsequenz gemäß Anspruch 5, welche ein HPV-Polypeptid eines der Typen 6, 11, 16, 18, 33 oder 45 codiert, oder eine Fusion aus zwei oder mehr Polypeptiden, von einem oder mehreren HPV-Virustypen 6, 11, 16, 18, 33 oder 45.

7. Polynucleotidsequenz gemäß Anspruch 5, welche ein HPV-Polypeptid eines HPV-Typs oder eines Subtyps codiert, der ausgewählt ist aus HPV 11, 6a oder 6b.

8. Polynucleotidsequenz gemäß irgendeinem der vorhergehenden Ansprüche, welche ein mutiertes HPV E1-Polypeptid mit den folgenden Mutationen K83G, R84G und G483D codiert.

9. Polynucleotidsequenz gemäß irgendeinem der Ansprüche 1 bis 7, welche ein mutiertes HPV E2-Polypeptid codiert, das eine KIIIA-Mutation umfasst.

10. Polynucleotidsequenz gemäß irgendeinem der vorhergehenden Ansprüche, wobei das codierte HPV-Polypeptid das Ganze oder einen Teil eines HPV-"early"-Genprodukts umfasst.

11. Polynucleotidsequenz gemäß Anspruch 9, wobei das codierte HPV-Polypeptid das Gesamte oder einen Teil von E1 oder E2 umfasst, oder eine Fusion aus dem Gesamten oder einem Teil von E1 oder E2 mit einem anderen HPV-Polypeptid.

12. Polynucleotidsequenz gemäß irgendeinem vorhergehenden Anspruch mit einem Codon-Verwendungskoeffizienten für E. coli-Gene von mehr als 0,6.

13. Polynucleotidsequenz wie in (SEQ ID NO: 10, Fig. 5) dargestellt, oder ein Fragment oder ein Analogon davon, welches die Codon-Verwendung davon bewahrt.

14. Polynucleotidsequenz wie in (SEQ ID NO: 11, Fig. 6) dargestellt, oder ein Fragment oder ein Analogon davon, welches die Codon-Verwendung davon bewahrt.

15. Expressionsvektor, umfassend eine Polynucleotidsequenz gemäß irgendeinem vorhergehenden Anspruch, operativ an eine Kontrollsequenz gebunden, welche in der Lage ist, die Expression der Polynucleotidsequenz durch eine Wirtszelle zu bewirken.

16. Expressionsvektor gemäß Anspruch 15, welche in der Lage ist, die Expression der Polynucleotidsequenz in bakteriellen, Insekten- oder Säugerzellen zu bewirken.

17. Expressionsvektor gemäß Anspruch 15 oder Anspruch 16, worin der Vektorhintergrund p7313PLc ist, wie in Fig. 7 gezeigt.

18. Wirtszelle, umfassend eine Polynucleotidsequenz gemäß irgendeinem der Ansprüche 1 bis 14.

19. Wirtszelle, umfassend einen Expressionsvektor gemäß irgendeinem der Ansprüche 15 bis 17.

20. Wirtszelle gemäß Anspruch 18 oder Anspruch 19, welche eine bakterielle, Säuger- oder Insektenzelle ist.

21. Pharmazeutische Zusammensetzung, umfassend eine Polynucleotidsequenz gemäß irgendeinem der Ansprüche 1 bis 14.

22. Pharmazeutische Zusammensetzung, umfassend einen Vektor gemäß irgendeinem der Ansprüche 15 bis 17.

23. Pharmazeutische Zusammensetzung gemäß Anspruch 21 oder Anspruch 22, umfassend eine Vielzahl an Partikeln, bevorzugt Goldpartikel, die mit DNA beschichtet sind.

24. Pharmazeutische Zusammensetzung gemäß Anspruch 22, umfassend einen pharmazeutisch annehmbaren Exzipienten und den DNA-Vektor.

25. Pharmazeutische Zusammensetzung gemäß irgendeinem der Ansprüche 21 bis 24, weiter umfassend ein Adjuvans.

26. Pharmazeutische Zusammensetzung gemäß Anspruch 25, wobei das Adjuvans als eine Fusion mit dem HPV-Polypeptid, das durch das Polynucleotid codiert wird, codiert ist.

27. Verwendung eines Polynucleotids gemäß irgendeinem der Ansprüche 1 bis 14 zur Herstellung eines Medikaments zur Behandlung oder Prophylaxe einer HPV-Infektion.

28. Verwendung eines Vektors gemäß irgendeinem der Ansprüche 15 bis 17 zur Herstellung eines Medikaments zur Behandlung oder Prophylaxe einer HPV-Infektion.

29. Verwendung gemäß irgendeinem der Ansprüche 27 und 28, wobei die HPV-Infektion eine Infektion von einem oder mehreren der HPV-Typen 6, 11, 16 oder 18 ist.

30. Verwendung eines Polynucleotids gemäß irgendeinem der Ansprüche 1 bis 14, eines Vektors gemäß irgendeinem der Ansprüche 15 bis 17 zur Herstellung eines Medikaments zur Behandlung oder Prophylaxe kutaner (Haut-) Warzen, Genitalwarzen, atypischer schuppenartiger Zellen von unbestimmter Bedeutung (ASCUS), Gebärmutterhalsdysplasie, intraepitheliale Gebärmutterhalsneoplasie (CIN) oder Gebärmutterhalskrebs.

## Revendications

1. Séquence de polynucléotide qui code pour une séquence d'acides aminés du virus du papillome humain d'un antigène précoce du VPH ou un fragment de celle-ci, ledit polynucléotide étant capable d'induire une réponse immunitaire lorsqu'il est administré *in vivo*, **caractérisée en ce que** ledit polynucléotide présente un coefficient d'usage de codons supérieur à 0,5 mais inférieur à 1,0 pour un gène humain fortement exprimé.

2. Séquence de polynucléotide selon la revendication 1 qui est une séquence d'ADN.

3. Séquence de polynucléotide selon la revendication 1 ou 2 qui code pour un polypeptide du VPH d'un type ou sous-type du VPH associé au cancer du col de l'utérus, aux verrues cutanées bénignes ou aux verrues génitales.

4. Séquence de polynucléotide selon la revendication 3 qui code pour un polypeptide du VPH de l'un des types suivants : 1-4, 6, 7, 10, 11, 16, 18, 26-29, 31, 33, 35, 39, 49, 51, 52, 56, 58, 59 et 68.

5. Séquence de polynucléotide selon la revendication 4 qui code pour un polypeptide du VPH d'un type ou sous-type du VPH qui est associé au cancer du col de l'utérus ou aux verrues génitales.

6. Séquence de polynucléotide selon la revendication 5 qui code pour un polypeptide du VPH de l'un des types suivants : 6, 11, 16, 18, 33 ou 45 ; ou pour une fusion de deux polypeptides ou davantage d'un ou davantage des virus VPH des types 6, 11, 16, 18, 33 ou 45.

7. Séquence de polynucléotide selon la revendication 5 qui code pour un polypeptide du VPH d'un type ou sous-type du VPH choisi parmi le VPH 11, 6a ou 6b.

8. Séquence de polynucléotide selon l'une quelconque des revendications précédentes qui code pour un polypeptide E1 muté du VPH comportant les mutations suivantes : K83G, R84G et G483D.

9. Séquence de polynucléotide selon l'une quelconque des revendications 1 à 7 qui code pour un polypeptide E2 muté du VPH comportant une mutation KIIIA.

10. Séquence de polynucléotide selon l'une quelconque des revendications précédentes dans laquelle le polypeptide codé du VPH comprend la totalité ou une partie d'un produit d'un gène précoce du VPH.

11. Séquence de polynucléotide selon la revendication 9 dans laquelle le polypeptide codé du VPH comprend la totalité ou une partie de E1 ou E2, ou une fusion de la totalité ou d'une partie de E1 ou E2 avec un autre polypeptide du VPH.

12. Séquence de polynucléotide selon l'une quelconque des revendications précédentes présentant un coefficient d'usage de codons pour les gènes de *E. coli* supérieur à 0,6.

13. Séquence de polynucléotide telle que présentée dans la (SEQ ID N° 10, Fig. 5), ou fragment ou analogue de celle-ci qui conserve l'usage de codons de celle-ci.

14. Séquence de polynucléotide telle que présentée dans la (SEQ ID N° 11, Fig. 6), ou fragment ou analogue de celle-ci qui conserve l'usage de codons de celle-ci.

15. Vecteur d'expression comprenant une séquence de polynucléotide selon l'une quelconque des revendications précédentes fonctionnellement reliée à une séquence de contrôle qui est capable de se charger de l'expression de la séquence de polynucléotide par une cellule hôte.

16. Vecteur d'expression selon la revendication 15 qui est capable de diriger l'expression de la séquence de polynucléotide dans des cellules bactériennes, d'insecte ou de mammifère.

17. Vecteur d'expression selon la revendication 15 ou la revendication 16 dans lequel le squelette du vecteur est p7313PLc comme il est montré à la figure 7.

18. Cellule hôte comprenant une séquence de polynucléotide selon l'une quelconque des revendications 1-14.

19. Cellule hôte comprenant un vecteur d'expression selon l'une quelconque des revendications 15-17.

20. Cellule hôte selon la revendication 18 ou la revendication 19 qui est une cellule bactérienne, de mammifère ou d'insecte.

21. Composition pharmaceutique comprenant une séquence de polynucléotide selon l'une quelconque des revendications 1-14.

22. Composition pharmaceutique comprenant un vecteur selon l'une quelconque des revendications 15-17.

23. Composition pharmaceutique selon la revendication 21 ou la revendication 22 comprenant une pluralité de particules, de préférence des particules d'or, enrobées d'ADN.

24. Composition pharmaceutique selon la revendication 22 comprenant un excipient pharmaceutiquement acceptable et le vecteur à ADN.

25. Composition pharmaceutique selon l'une quelconque des revendications 21-24 comprenant en outre un adjuvant.

26. Composition pharmaceutique selon la revendication 25 dans laquelle l'adjuvant est codé sous forme de fusion avec le polypeptide du VPH codé par le polynucléotide.

27. Utilisation d'un polynucléotide selon l'une quelconque des revendications 1-14 dans la fabrication d'un médicament pour le traitement ou la prophylaxie d'une infection due au VPH.

28. Utilisation d'un vecteur selon l'une quelconque des revendications 15-17 dans la fabrication d'un médicament pour le traitement ou la prophylaxie d'une infection due au VPH.

29. Utilisation selon l'une quelconque des revendications 27 et 28 dans laquelle l'infection due au VPH est une infection due à l'un ou plusieurs d'entre les VPH de types 6, 11, 16 ou 18.

30. Utilisation d'un polynucléotide selon l'une quelconque des revendications 1-14, d'un vecteur selon l'une quelconque des revendications 15-17, dans la fabrication d'un médicament pour le traitement ou la prophylaxie de verrues cutanées (de la peau), de verrues génitales, de cellules malpighiennes atypiques de signification indéterminée (ASCUS), de dysplasies cervicales, de néoplasies cervicales intraépithéliales (CIN) ou du cancer du col de l'utérus.
